# EUROPEAN PATENT APPLICATION

(11) **EP 4 382 042 A1**
(43) Date of publication of application: **12.06.2024**
(21) Application number: 22211704.6
(22) Date of filing: 06.12.2022
(51) Int. Cl.: A61B 5/06, G01R 33/00

(54) **INCREASING SIGNAL-TO-NOISE RATIO OF MINIATURE MAGNETO-MECHANICAL RESONATORS**

(71) Applicant: Koninklijke Philips N.V., 5656 AG Eindhoven (NL)
(72) Inventor: GLEICH, Bernhard, Eindhoven (NL); RAHMER, Jürgen Erwin, Eindhoven (NL)
(74) Representative: Philips Intellectual Property & Standards

(57) **Abstract**

The present invention relates to a passive medical sensing device for at least one of sensing or position tracking. The device comprises a housing and a plurality of magneto-mechanical resonators arranged in the housing, wherein at least one of the plurality of magneto-mechanical resonators is magnetically coupled to at least another of the plurality of magneto-mechanical resonators. At least one of the magneto-mechanical resonators comprises at least one movable magnet mounted to the housing and configured to rotate around a rotational axis, and a restoring unit configured for exerting a restoring force on the at least one movable magnet when the movable magnet is deflected from a rest position of the movable magnet with respect to the rotational axis.

## Description

### FIELD OF THE INVENTION

The present invention relates to a passive medical sensing device (-s) for at least one of sensing or position tracking, an arrangement comprising the probe with the passive medical sensing device (-s), and a method / methods of sensing or tracking of a position of a probe comprising the passive medical sensing device.

### BACKGROUND OF THE INVENTION

Magneto-mechanical resonators (MMRs) are a new and promising way to generate small markers and sensors for medical applications. They can comprise tiny magnets that resonate in the presence of a second magnet and can be inserted within a probe into a subject, such as a human. If excited by an external electro-magnetic pulse, they can oscillate and emit electro-magnetic radiation with a resonance frequency that can be measured. From the received oscillating signal, it is possible to determine a position of the magneto-mechanical resonator in the subject. Further, MMRs can be used as marker for in-body sensing applications and can measure, for example, pressure or temperature.

However, one of the main technical challenges in the tiny markers is the insufficient signal-to-noise (SNR) ratio. This ratio determines the minimum possible size of the MMR for a given detection distance. Many applications would benefit from an improved signal.

### SUMMARY OF THE INVENTION

The inventors of the present invention have thus found that it would be advantageous to have a device which provides magneto-mechanical resonators with an improved signal-to-noise ratio while keeping the size of the device small

It is an object of the present invention to provide an improved device comprising magneto-mechanical resonators that provides an increased signal-to-noise ratio such that the tracking distance may be increased considerably without significantly increasing the size of the device, allowing for better integration into the clinical workflow.

The object of the present invention is solved by the subject matter of the independent claims, wherein further embodiments are incorporated in the dependent claims.

The described embodiments similarly pertain to the passive medical sensing device for at least one of sensing or position tracking, the arrangement comprising the probe with the passive medical sensing device for at least one of sensing or position tracking, and the method of sensing or tracking of a position of a probe comprising the passive medical sensing device. "Passive" within the context of the present invention may mean that it is measuring the signals in a human body in a certain period. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first aspect of the invention, there is provided a passive medical sensing device for at least one of sensing or position tracking. The device comprises a housing and a plurality of magneto-mechanical resonators arranged in the housing, wherein at least one of the plurality of magneto-mechanical resonators is magnetically coupled to at least another of the plurality of magneto-mechanical resonators. At least one of the magneto-mechanical resonators comprises at least one movable magnet mounted to the housing and configured to rotate around a rotational axis, and a restoring unit configured for exerting a restoring force on the at least one movable magnet when the movable magnet is deflected from a rest position of the movable magnet with respect to the rotational axis.

By providing a plurality of magneto-mechanical resonators arranged such that the resonators are magnetically coupled, and that at least some of them can oscillate in unison when excited by an external field. "In unison" within the context of the present invention may mean that at least some MMR's are synchronized in their oscillation within a certain time period. The emitted signal and thus the signal-to-noise ratio from the magneto-mechanical device according to the invention can be improved. Preferably, a fraction of more than 50% of the resonators is magnetically coupled to at least one other resonator. Resonators not-coupled to other resonators may be used for a second type of measurement provided by the device, or as a reference for another measurement, for example. Preferably, each of the of the plurality of magneto-mechanical resonators is magnetically coupled to at least another of the plurality of magneto-mechanical resonators, and/or each of the magneto-mechanical resonators comprises at least one movable magnet and a restoring unit.

A space in a probe, for example, that is available to be filled with a resonating magneto-mechanical device can thus be sub-divided into different compartments that are each filled with at least one micro-magnetic resonator. Alternatively, the magneto-mechanical resonators can be positioned next to one another in the available space, like, for example, the housing of the device. The movable magnet can be provided as a permanent magnetic oscillator.

Therefore, the available space is divided into smaller sub-spaces thus allowing to employ more than one MMR in that available space. The gain in the signal quality and the possible detection distance comes from the scaling law of the signal-to-noise ratio (SNR) in the magneto-mechanical resonators. The SNR increases with the power of 7/3 with respect to linear dimensions of the magneto-mechanical resonator, while volume increases with the 3rd power. The core reason why smaller MMRs perform better is that their moment of inertia is relatively low. So, increasing the linear dimension of the magneto-mechanical resonator 10-fold increases the SNR only 215-fold instead of the 1000-fold increase of the volume. However, 1000 individual MMRs in that volume would increase the SNR 1000-fold, provided all MMRs would oscillate in unison as they are synchronized. This is achieved by bringing the MMRs close to each other such that the MMRs are magnetically coupled. Mechanical coupling of the MMRs may be possible, too, but is less efficient and more difficult to achieve. Thus, one advantage of the present invention is the improvement of the signal-to-noise ratio of the magneto-mechanical device for marking or sensing compared to the state of the art where only one magneto-mechanical resonator is provided in a comparable available volume.

This device according to the invention is especially useful in non-single-use items, such as surgical devices or imaging devices such as ultrasound probes. Due to the improved SNR, the tracking distance may be increased considerably, allowing for better integration into clinical workflow and better clinical results to be achieved.

In an embodiment of the invention, the restoring unit configured for exerting the restoring force is a restoring magnet. Thus, the restoring magnet exerts a magnetic field at the position of the movable magnet such that the movable magnet experiences a force due to its magnetic dipole moment that rotates the movable magnet into the rest position where the magnetic dipole moment is oriented in the direction of the magnetic field of the restoring magnet. Preferably, the magnetic moment of the restoring magnet and the magnetic moment of the movable magnet are aligned antiparallel. The restoring magnet can be, for example, a permanent magnet.

In the preferred embodiment, the restoring force is enacted due to a magnetic force of the restoring magnet. Other embodiments are possible within the context of the present invention. In some examples it is beneficial also to include springs, string or other suspension devices. In a non-limiting example, a spring is used. Usage of a spring is beneficial due to the fact that the movable magnets are preferably suspended from strings attached to the housing.,. In some embodiments, the spring or filament itself may act as torsion spring. As an example, the filament may contribute by about 10% to the overall restoring torque. However, alternatively, more than one mechanical spring can be provided in the resonators, and it may even be possible to use only a mechanical spring without a restoring magnet.

In an embodiment of the invention, the restoring magnet is fixed to the housing such that no rotational movement of the restoring magnet is allowed. Thus, the magnetic field produced by the restoring magnet is constant in time and orientation, and the restoring force exerted on the movable magnet is solely dependent on the deflection of the movable magnet. However, as an alternative, it is possible to provide a restoring magnet that is not fixed to the housing and may be also mounted such that a rotation of the restoring magnet is permitted. In this embodiment, both magnets may oscillate and influence each other in order to define a rest position.

Preferably, the movable magnets are attached to the housing via a spring, a filament, or a thread or in any other way, and the restoring magnets are fixedly attached to the housing. Each resonator may be provided as sub-unit comprising at least a partial housing. This partial housing does not need to be a full enclosure, but it may provide a fixed string attachment point relative to the fixed magnet. As these sub-units do not need to be pre-assembled during an assembly operation, the movable and fixed magnets may also be assembled in a pre-made housing, or at least a mechanical structure that offers the filament attachment points without being a full cover. Any arbitrary relationship number between fixed magnets and movable magnets can be provided. In case pre-assembled resonators are combined in the production process, they can be fixed together by gluing, soldering, welding, or by juts that have some shapes that attach to each other and engage like bricks. Alternatively, the movable and fixed magnets can be assembled in a common housing by gluing them in place. As magnetic forces are acting, magnet shapes that lock the orientation of the magnets when inserted into the housing may be provided. Cubic magnets may be easier to hold in place than spherical magnets, and tight-fitting canals to insert them into the housing may be provided. Alternatively, a set of magnetic tools that hold the magnets in the right orientation before the glue cures can be provided, optionally multiple tools and multiple gluing steps may be necessary. For the movable magnets, the use of a magnetic tool seems to be a preferable option, especially if no fixed magnets are provided.

In an embodiment of the invention, the restoring unit is a restoring magnetic object, the restoring magnetic object being coupled to the housing and coupled via a magnetic field to a movable magnetic object from the plurality of magneto-mechanical resonators, the coupling being such that the restoring magnetic object exerts a restoring force on the coupled movable magnetic object when the coupled movable magnetic object is rotated from its rest position.

In an embodiment of the invention, a magnetic moment of each of the restoring magnets of the plurality of magneto-mechanical resonators is arranged in parallel with a magnetic moment of a restoring magnet of a respective neighboring magneto-mechanical resonator, and a magnetic moment of each of the movable magnets of the plurality of magneto-mechanical resonators is arranged in parallel with a magnetic moment of a movable magnet of the respective neighboring magneto-mechanical resonator when the movable magnets are in their respective rest position. Thus, the restoring magnets are arranged such that the magnetic dipole moments of all of the restoring magnets point in a first direction, wherein the movable magnets are arranged such that in the rest position of the movable magnets the magnetic dipole moments of all of the movable magnets point in a second direction. Preferably, the first direction and the second direction are antiparallel to each other. The plurality of the magneto-mechanical resonators may be arranged next to each other in a line such that the first direction and the second direction is perpendicular to a direction of the line. Alternatively, the plurality of the magneto-mechanical resonators may be arranged such that the first direction and the second direction is parallel or antiparallel, respectively, with respect to a direction of the line.

In an embodiment of the invention, a magnetic moment of each of the restoring magnets of the plurality of magneto-mechanical resonators is arranged antiparallel to a magnetic moment of a restoring magnet of a respective neighboring magneto-mechanical resonator, and a magnetic moment of each of the movable magnets of the plurality of magneto-mechanical resonators is arranged antiparallel to a magnetic moment of a movable magnet of the respective neighboring magneto-mechanical resonator when the movable magnets are in their respective rest position. Thus, the restoring magnets of the plurality of magneto-mechanical resonators may be arranged such that the magnetic dipole moment of one of the restoring magnets points in the opposite direction as the magnetic dipole moments of the restoring magnets of the neighboring magneto-mechanical resonators. Thus, the magneto-mechanical resonators may be arranged in a line, for example, with an alternating direction of the magnetic dipole moments of the restoring magnets along the line. In addition, a direction of the magnetic dipole moment of the movable magnet of each of the magneto-mechanical resonators may be opposite to a direction of the magnetic dipole moment of the adjacent magneto-mechanical resonators.

Preferably, the direction of the movable magnet is antiparallel to the direction of the restoring magnet for each of the magneto-mechanical resonators. In one embodiment, the magneto-mechanical resonators may be arranged in a line such that the directions of the movable magnet and the restoring magnets of all of the plurality of magneto-mechanical resonators are perpendicular to a direction of the line. In an alternative embodiment, the magneto-mechanical resonators may be arranged in a line such that the directions of the movable magnet and the restoring magnets of all of the plurality of magneto-mechanical resonators are parallel or antiparallel to the direction of the line.

In an embodiment of the invention, the movable magnet is located on the rotational axis, and/or the rotational axis is perpendicular, or at least substantially perpendicular, to a magnetic dipole moment of the movable magnet. Substantially perpendicular has to be understood as perpendicular with an angular deviation from a 90° angle of less than 45°, preferably less than 20 °, even more preferably less than 10°. Thus, the rotational axis of each of the movable magnets of the plurality of magneto-mechanical resonators may be arranged such that it goes directly through a center of the magnetic dipole moment of the movable magnet. The axis of rotation may be substantially perpendicular to the direction of the magnetic dipole moment of the movable magnet for each of the plurality of magneto-mechanical resonators in order to maximize a change of the magnetic field produce by the movable magnet when oscillating.

In an embodiment of the invention, the movable magnet is configured to perform an oscillating rotational movement with a frequency f₀ when the movable magnet is deflected out of the rest position. Thus, a variation of an external electro-magnetic field like, for example, a short pulse may deflect the movable magnet from its rest position. As the restoring unit for exerting a restoring force forces the movable magnet back to its rest position, an oscillation of the movable magnet around its rest position with a characteristic frequency f₀ is excited.

In an embodiment of the invention, each of the plurality of magneto-mechanical resonators, when the movable magnet oscillates around the rest position, is configured to effect a change of the magnetic field at the position of the movable magnet of the at least another of the plurality of magneto-mechanical resonators in a direction perpendicular to both the rotational axis and the magnetic dipole moment of the movable magnet of the at least another of the plurality of magneto-mechanical resonators that is larger than 1 µT, preferably larger than 10 µT, more preferably larger than 100 µT, thus magnetically coupling the magneto-mechanical resonator with the at least another of the plurality of magneto-mechanical resonators. In order to provide an arrangement of the plurality of magneto-mechanical resonators oscillating in unison, the magneto-mechanical resonators need to be magnetically coupled. This may be achieved, for example, by adjusting the distance between neighboring magneto-mechanical resonators, or by increasing the magnetic field strength produced by each of the magneto-mechanical resonators. In order to oscillate in unison, each of the plurality of magneto-mechanical resonators may be coupled to each of the other of the plurality of magneto-mechanical resonators. However, it may be sufficient to provide an arrangement of the magneto-mechanical resonators, where each of the plurality of magneto-mechanical resonators is magnetically coupled to at least one neighboring magneto-mechanical resonator. Thus, a grid may be provided, where a first resonator is coupled to a second resonator, and where the second resonator is coupled to a third resonator, thus providing an indirect coupling of the first resonator to the third resonator, such that all resonators may oscillate in unison when excited. In order to achieve magnetic coupling between two magneto-mechanical resonators, the change of the magnetic field produced by the dynamic dipole moment of the first magneto-mechanical resonator during oscillation needs to exceed a certain limit at the position of the second magneto-mechanical resonator, in a direction perpendicular to a direction of the magnetic dipole moment of the movable magnet of the second magneto-mechanical resonator in the rest position of the movable magnet of the second resonator. If this dynamic field change exceeds a limit of 1 µT, preferably 10 µT, even more preferably 100 µT, the two magneto-mechanical resonators may be regarded as magnetically coupled in the context of the invention. This coupling ensures that all of the coupled magneto-mechanical resonators oscillate in unison and thus add their oscillating magnetic dipole moments.

In an embodiment of the invention, the plurality of magneto-mechanical resonators is arranged such that each of the plurality of magneto-mechanical resonators is coupled to any another of the plurality of magneto-mechanical resonators magnetically either directly or indirectly. Thus, two adjacent magneto-mechanical resonators may be magnetically coupled if the dynamic field of the first resonator exceeds a certain limit at the position of the second resonator, and vice versa, as already explained. This is understood as a direct magnetic coupling. However, it may be sufficient if two magneto-mechanical resonators are indirectly magnetically coupled via a chain of directly magnetically coupled magneto-mechanical resonators. This may also result in a grid of a plurality of magneto-mechanical resonators oscillating in unison. In case always only pairs of two resonators are magnetically coupled, the pair of resonators would provide the SNR gain, but not the whole assembly. Thus, for more than two resonators in the plurality of resonators, at least some of the resonators may need to be magnetically coupled to two other resonators. If always each resonator is coupled to only two resonators, a "line" of coupling through the device may be achieved. However, preferably, a resonator is coupled to at least all neighboring resonators and even more preferably additionally also to the next but one neighbors.

In an embodiment of the invention, a resonance frequency f of the device differs from the resonance frequency *f₀* of each of the plurality of magneto-mechanical resonators. The plurality of magnetically coupled magneto-mechanical resonators may oscillate with a resonance frequency *f* that differs from the characteristic resonance frequency *f₀* of a single magneto-mechanical resonator. This may be due to the magnetic coupling and mutual influences of the movable magnets and the restoring magnets of neighboring magneto-mechanical resonators. The resonance frequency *f* of the device may depend on an external magnetic field applied parallel or antiparallel to the magnetic moments of the movable magnets. Usually, the frequency *f* is 5% to 20% higher or lower than the single resonator frequency *f₀.* However, it may be possible to provide a device having the same frequency *f* as *f₀* or a frequency f similar to *f₀.*

In an embodiment of the invention, a length of the movable magnets of the plurality of magneto-mechanical resonators in the direction of the rotational axis is larger than a length of the movable magnets perpendicular to the rotational axis, and/or each of the of the plurality of magneto-mechanical resonators comprises a collector of a soft magnetic material arranged adjacent to the movable magnet and configured for focusing a magnetic field applied to or emitted from the movable magnet. As it may be quite expensive to have a plurality of individual resonators combined, some strategies to reduce the number can be employed. To reduce cost, additional strategies are disclosed that may reduce the number of resonators employed while maintaining the signal-to-noise benefit. Alternatively, these strategies may allow to further increase the signal-to-noise ratio. As already explained, the core reason why smaller MMRs perform better with respect to the signal-to-noise ratio in relation to the absolute signal height is that their moment of inertia is relatively low. If the size of an object is scaled linearly in all directions by a predetermined factor, the moment of inertia scales with the fifth power of the factor, while the volume scales only with the third power of this factor. However, if, for example, a cylinder is lengthened along its rotational axis, the moment of inertia scales the same as the volume and hence, the SNR increases with the volume without the deteriorating effect of the heavily increasing moment of inertia. Thus, providing an elongated moveable magnet in the magneto-mechanical resonator may increase the signal-to-noise ratio. In addition or alternatively, the device and/or the magneto-mechanical resonators can be provided with a collector made of a magnetically soft material arranged next to the movable magnets of the plurality of magneto-mechanical resonators in order to concentrate or focus the field applied externally to the resonators, and in particular to concentrate and/or amplify the magnetic field emitted by the oscillating movable magnet. For example, the collector can be made of iron as an example of a soft magnetic material that may amplify and concentrate magnetic fields. A multitude of soft magnetic materials can be used for focusing the magnetic field. Preferably, soft ferrites, which are available on the market, can be utilized due to their low hysteresis losses. A high saturation magnetization can be beneficial, although not necessary. Alternatively, soft magnetic metallic glasses and the high permeability nickel-iron alloys like permalloy or mu-metal can be used. High purity iron, nickel or cobalt can be used as well. Also highly paramagnetic materials like, for example, Gadolinium slightly above its curie temperature of 19°C can be used as soft magnetic material in the context of the invention. Other materials having similar properties may also be used.

In an embodiment of the invention, the plurality of magneto-mechanical resonators is arranged in a one-dimensional, a two-dimensional or a three-dimensional grid. There are many ways in which the plurality of magneto-mechanical resonators can be arranged in the available space. For example, the MMRs may be arranged in a linear, one-dimensional configuration. However, the plurality of magneto-mechanical resonators may preferably be arranged in a two-dimensional or three-dimensional grid to provide a better coverage of the available space and/or in order to improve the magnetic coupling. It is also possible to employ the resonators in an arbitrary configuration to improve the magnetic coupling. Any grid or crystal structure known in crystallography may be used to arrange the magneto-mechanical resonators. Alternatively, a disordered grid may be provided.

In an embodiment of the invention, the device comprises a termination magnet attached to the housing and configured for correcting edge effects of the plurality of magneto-mechanical resonators, and/or at least one of the of the plurality of magneto-mechanical resonators that is arranged at a border area of the of the plurality of magneto-mechanical resonators comprises a movable magnet that is spatially fixed in order to correct for edge effects of the plurality of magneto-mechanical resonators.

One important issue with respect to a device according to the invention is the termination of the MMRs in the boundary region of the arrangement, for example of the lattice. The various magnets generate forces on the other magnets that may be suspended from threads, for example. As the magnets in the boundary region experience asymmetric forces due to their location, this may cause a displacement of the movable magnets from the position they would have relative to a fixed restoring magnet in a single MMR configuration. This displacement may be detrimental to the function of the assembly. However, deep inside the assembly of a periodic lattice, the additional forces may cancel and therefore the operation of the device is unimpeded. At the border or edges of the arrangement of MMRs, this may not apply.

Therefore, as a solution, the border MMRs, i.e. the resonators that are positioned in a boundary area of the grid of MMRs and that experience asymmetric force due to their position within the grid, may be prevented from being mobile. For example, the movable magnet may be fixed with respect to at least a displacement, while a rotational movement may still be allowed. Alternatively, it may be possible to prevent the magnets at the border region even from rotating. This fixation may be achieved, for example, by gluing the oscillating magnet in place. As the magnetic forces of a magnetic dipole fall off rapidly, fixing one or two layers of the resonators may be sufficient.

Alternatively, there may be other solutions to correct for edge effects. As there is a multitude of possible magnet assemblies that can be arranged outside of the arrangement of the MMRs, or outside of the lattice, it is possible to provide at least one termination magnet that is located next to the arrangement of the plurality of magneto-mechanical resonators in order to compensate the forces of the asymmetric arrangement of the MMRS and thus solve the problem of the edge effects. This termination magnet can be provided in a way that its magnetic field compensates for the magnetic field at the position of border MMRs that is missing due to the absence of in infinite grid of MMRs, thus terminating the grid of MMRs. By numerical optimization of magnet positions and sizes to minimize magnets displacements, it is possible to determine a solution how a termination magnet may be positioned, while allowing only a minimum amount of magnetic material, a minimum number of objects, a minimum occupied space or similar criteria.

Finding of the correct position for fixing the termination magnets and/or the movable magnets is preferably done with a simulation software. The number of end magnets or termination magnets can be arbitrarily defined, preferably one termination magnet per facing resonator. Then a volume can be defined in which the magnet is allowed to be placed. By variation of size, position and orientation of the magnet, a useful measure like, for example, the RMS movable magnet deviation from its ideal rest position can be minimized.

According to another aspect of the invention, there is provided an arrangement comprising a probe with a device according to any of the preceding embodiments, an emitter configured for emitting a pulse of an electro-magnetic radiation for deflecting the movable magnets of the plurality of magneto-mechanical resonators of the device out of the rest position of the movable magnets and for exciting an oscillating movement of the movable magnets with a resonance frequency f of the device, a receiver configured for receiving an electro-magnetic field emitted by the movable magnets of the magneto-mechanical resonators of the device oscillating with the resonance frequency *f,* and a controller configured for determining a position and/or an orientation of the device based on the received electro-magnetic field emitted by the movable magnets of the magneto-mechanical resonators of the device oscillating with the resonance frequency *f.* With this arrangement, it is possible to determine the position of a probe inserted into a subject.

According to another aspect of the invention, there is provided the use of an arrangement according to any of the preceding embodiments for determining a position and/or an orientation of a probe comprising a device according to any of the preceding embodiments inserted into a subject.

According to another aspect of the invention, better SNR allows reduce the size of the MMRs and/or achieve a larger detection distance.

According to another aspect of the invention, there is provided a method of sensing or tracking of a position of a probe comprising a passive medical sensing device according to any of the preceding embodiments. The method comprises the steps of providing a probe comprising the passive medical sensing device, inserting the probe into an object, and emitting a pulse of an electro-magnetic radiation for deflecting the movable magnets of the plurality of magneto-mechanical resonators of the device out of the rest position of the movable magnets and for exciting an oscillating movement of the movable magnets with a resonance frequency *f* of the device. The method further comprises the steps of receiving an electro-magnetic field emitted by the movable magnets of the magneto-mechanical resonators of the device oscillating with the resonance frequency *f*, and determining a position and/or an orientation of the device based on the received electro-magnetic field emitted by the movable magnets of the magneto-mechanical resonators of the device oscillating with the resonance frequency *f.* This method increases favorably the signal-to-noise ratio of the magnetic field received from the plurality of magneto-mechanical resonators.

According to another aspect of the invention, there is provided a computer program comprising instructions which, when the program is executed by a computer, cause the computer to carry out the steps of the method according to one of the preceding embodiments.

According to another aspect of the invention, there is provided a computer-readable storage medium comprising instructions which, when executed by a computer, cause the computer to carry out the steps of the method according to one of the preceding embodiments.

Thus, the benefits provided by any of the above aspects equally apply to all of the other aspects and vice versa.

The invention may relate to a passive medical sensing device for at least one of sensing or position tracking. The device comprises a housing and a plurality of magneto-mechanical resonators arranged in the housing, wherein at least one of the plurality of magneto-mechanical resonators is magnetically coupled to at least another of the plurality of magneto-mechanical resonators. At least one of the magneto-mechanical resonators comprises at least one movable magnet mounted to the housing and configured to rotate around a rotational axis, and a restoring unit configured for exerting a restoring force on the at least one movable magnet when the movable magnet is deflected from a rest position of the movable magnet with respect to the rotational axis.

The above aspects and embodiments will become apparent from and be elucidated with reference to the exemplary embodiments described hereinafter. Exemplary embodiments of the invention will be described in the following with reference to the following drawings.

### BRIEF DESCRIPTION OF THE DRAWINGS

Fig. 1 shows a schematic setup of a passive medical sensing device for at least one of sensing or position tracking according to an embodiment of the invention.
Figs. 2a to 2d show schematic setups of a passive medical sensing device for at least one of sensing or position tracking according to another embodiment of the invention.
Fig. 3 shows a schematic setup of a passive medical sensing device for at least one of sensing or position tracking comprising termination magnets according to another embodiment of the invention.
Figs. 4a and 4b show schematic setups of a passive medical sensing device for at least one of sensing or position tracking comprising a collector of a soft magnetic material and an elongated movable magnet according to another embodiment of the invention.
Fig. 5 shows a schematic setup of an arrangement according to another embodiment of the invention.

### DETAILED DESCRIPTION OF EMBODIMENTS

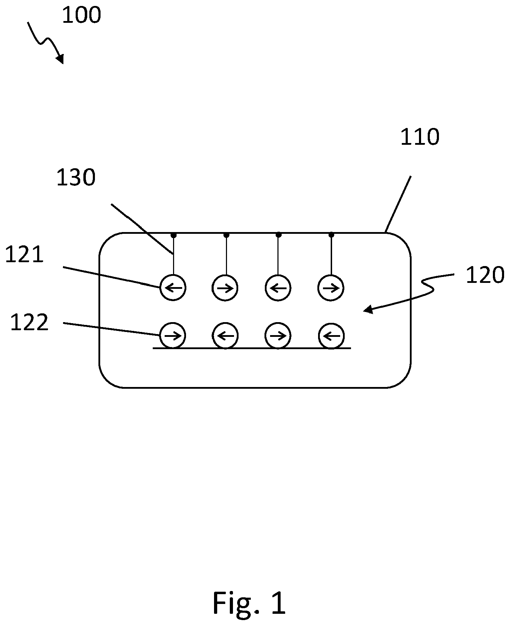

Fig. 1 shows a schematic setup of a passive medical sensing device 100 for at least one of sensing or position tracking according to an embodiment of the invention. The device 100 comprises a housing 110 in which a plurality of magneto-mechanical resonators 120 is arranged. The housing can be divided into several sub-spaces, with each sub-space being provided with one of the resonators. Each of the magneto-mechanical resonators 120 comprises a movable magnet 121, for example a permanent magnet, mounted to the housing 110 such that a rotational movement of the movable magnet 121 around a rotational axis 130 is allowed. For example, the movable magnet 121 can be suspended from a thread attached to the housing 110. The magnetic dipole moment of the movable magnet 121 is preferably orientated perpendicular to the rotational axis 130. A restoring unit for exerting a restoring force 122 is provided such that the movable magnet 121, if deflected from its rest position, is directed towards its rest position. Preferably, a position of the movable magnet 121 is fixed such that only a rotational movement around the rotational axis 130 is allowed. In combination with the restoring force, an oscillating movement of the movable magnet 121 can be performed. Preferably, the restoring unit for exerting a restoring force 122 can be a restoring magnet 123 like a permanent magnetic stator, which can be fixedly attached to the housing 110. The individual magneto-mechanical resonators 120 are arranged such that each of the plurality of magneto-mechanical resonators 120 is magnetically coupled to at least another of the neighboring magneto-mechanical resonators 120. The magneto-mechanical resonators 120 are preferably arranged such that each of the plurality of magneto-mechanical resonators 120 is magnetically coupled with any other of the plurality of magneto-mechanical resonators 120 either directly or indirectly via a chain of directly magnetically coupled magneto-mechanical resonators 120. With this coupling, it is ensured that all of the plurality of magneto-mechanical resonators 120 can oscillate in unison. In Fig 1, a 1-dimensional grid of MMRs is shown. However, it is possible to stack these linear assembly to planes and the planes to volumes in order to provide a two-dimensional or a three-dimensional arrangement. There are several modes possible how to stack the lines and the planes together to a three-dimensional grid. One example is to stack the magneto-mechanical resonators in a lattice with all oriented in the same direction.

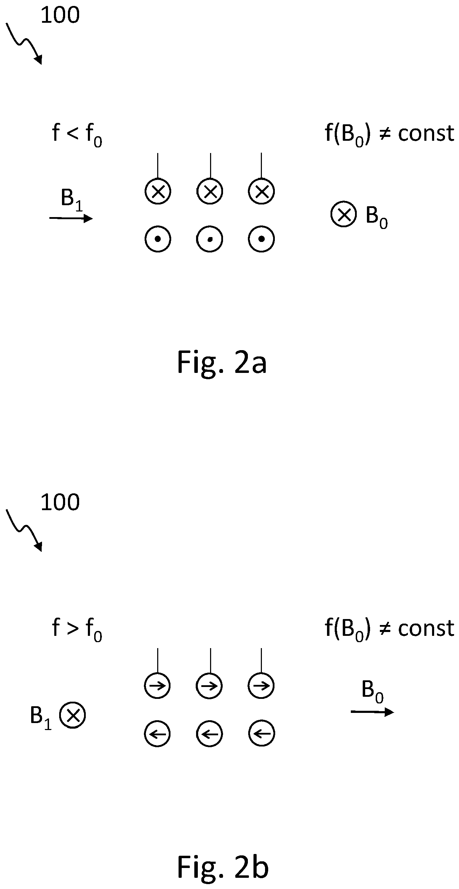

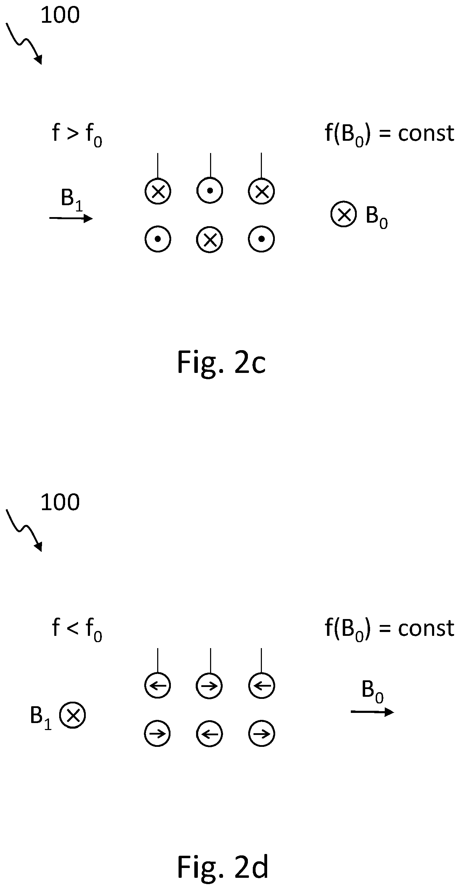

Figs. 2a to 2d show schematic setups of a passive medical sensing device 100 for at least one of sensing or position tracking according to another embodiment of the invention. These embodiments show different configurations of the arrangement of the magneto-mechanical resonators 120 according to the invention. The resonators each comprise in this embodiment a movable permanent magnet 121 movably mounted on a string shown in the upper row and fixed restoring magnets 123, which can be permanent magnets, in the lower row. Although the individual MMRs do not need a separate housing, a housing 110 around the whole assembly is advisable. In Fig 2a, the movable magnets 121 and the restoring magnets 123 are aligned in parallel, respectively, as their magnetic dipole moments are oriented in the same direction. This has the effect that the resonance frequency f of the assembly decreases over f₀ of a single MMR component and the whole system frequency depends on a perpendicular offset field B₀. If the two above-mentioned effects are positive or negative depends on the specific application. In some applications, the possibility to influence the resonance frequency by an external field may be desired. However, for sensor applications, external field effects are usually detrimental. Likewise, the signal-to-noise ratio may increase or decrease with increasing f, depending on other parameters and limiting factors. Fig 2b, 2c, and 2d depict other relative orientations of fixed restoring magnets 123 and oscillating movable magnets 123 together with the effect on the system resonance frequency f compared to the resonance frequency f₀, and the first order dependence of the resonance frequency f on an external static magnetic field, f(B₀), with the external magnetic field applied parallel to the magnetic dipole moment of the movable magnets 121. Preferably, all the individual MMR components should have a similar resonance frequency f₀.

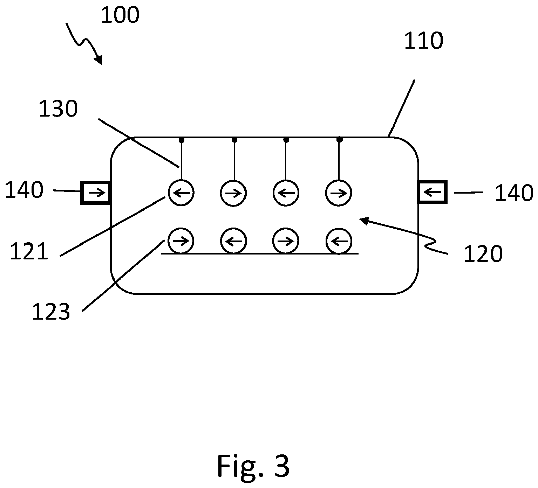

Fig. 3 shows a schematic setup of a passive medical sensing device 100 for at least one of sensing or position tracking comprising termination magnets 140 according to another embodiment of the invention. Two termination magnets are provided next to the arrangement of the plurality of magneto-mechanical resonators 120 and attached to the housing 110 in order to compensate the forces of the asymmetric arrangement of the MMRs and thus solve the problem of the edge effects.

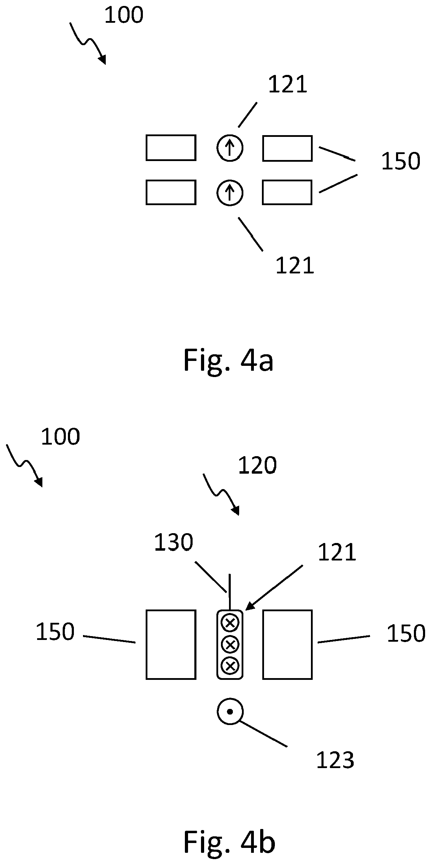

Figs. 4a and 4b show schematic setups of a passive medical sensing device 100 for at least one of sensing or position tracking comprising a collector of a soft magnetic material 150 and an elongated movable magnet 121 according to another embodiment of the invention. To achieve the goal of cost reduction, two methods are described in Fig 4a and 4b. One method depicted in Fig 4a is the use of a soft magnetic material that is provided in the form of a collector 150 next to the movable magnets 121. This collector concentrates the send and receive field of the magneto-mechanical resonators and increases the efficiency. This effect can also be used with a single MMR and is also useful in only one spatial dimension. Due to the demagnetizing fields of the soft magnetic material, the efficiency of this method may be lower than for the stacking of a plurality of MMRs, but the low-cost solution may be justified for some applications. However, if the arrangement of a plurality of resonators is combined with a collector of soft magnetic material, both effects can be advantageously combined. The second method shown in Fig 4b is to increase the length of the rotating movable magnet 121 suspending on the rotational axis 130. However, this cannot be done indefinitely as the attractive force to the static restoring magnet 123 will not be strong enough anymore. This method to increase the signal-to-noise ratio also works on a single MMR, but it is rare that additional space is only available in one dimension. If the maximum length of the rotating magnet still does not cover the available space, it is possible to stack multiple layers in this direction. Fig 4b shows a combination of an elongated movable magnet 121 with the use of collectors 150 made of a soft magnetic material like iron.

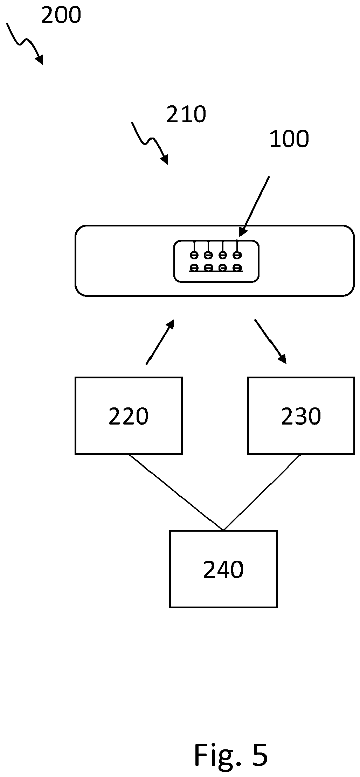

Fig. 5 shows a schematic setup of an arrangement 200 according to another embodiment of the invention. The arrangement comprises a probe 210 with a magneto-mechanical device 100 for marking or sensing, an emitter 220 for emitting a pulse of an electro-magnetic radiation for deflecting the movable magnets 121 of the plurality of magneto-mechanical resonators 120 out of the rest position and for exciting an oscillating movement of the movable magnets 121. The arrangement 200 further comprises a receiver 230 for receiving an electro-magnetic field emitted by the movable magnets 121 of the magneto-mechanical resonators 120 oscillating with the resonance frequency f, and a controller 240 for determining a position and/or an orientation of the device 100 based on the received electro-magnetic field emitted by the movable magnets 121 oscillating with the resonance frequency f.

First, from receive data, the system needs to identify if it is subject to interference from other systems. (This is described in a separate invention disclosure.)

In some embodiments, an analysis of interference situation is being made. Based on this, a list of interferers is generated, which includes the level (severity) of the interference. Once a certain, given (changeable) threshold is exceeded, the interferer shall be considered to be of relevance, and hence the following mitigation shall be applied to ensure continuous reliable operation of the at least one system:
- The first system sends a token to initiate synchronous operation.
- The second system replies to a token to confirm synchronization.
- The two systems intermittently send their Tx sequences, so that neither of them sends in the Rx phase of the other.

This simple description can be extended easily to more than 2 systems that need to be synchronized. Furthermore, the exchange of tokens can be realized by various methods: either using transmission via the magnetic communication path, i.e. the coils, transmitters and receivers intrinsic to the system, or via secondary communication paths, e.g. WLAN, Bluetooth or similar means. Besides initiating a synchronization, the tokens can carry various useful pieces of information, e.g. repetition time, received field strengths, etc.

The storage of the data coming from the computer device may be local (e.g., on the device itself) or on the cloud. When implemented as part of a cloud service or services, one or more computing devices may comprise an internal cloud, an external cloud, a private cloud, or a public cloud (e.g., commercial cloud). For instance, a private cloud may be implemented using a variety of cloud systems including, for example, Eucalyptus Systems, VMWare vSphere^{®}, or Microsoft^{®} HyperV. A public cloud may include, for example, Amazon EC2^{®}, Amazon Web Services^{®}, Terremark^{®}, Savvis^{®}, or GoGrid^{®}. Cloud-computing resources provided by these clouds may include, for example, storage resources (e.g., Storage Area Network (SAN), Network File System (NFS), and Amazon S3^{®}), network resources (e.g., firewall, load-balancer, and proxy server), internal private resources, external private resources, secure public resources, infrastructure-as-a-services (IaaSs), platform-as-a-services (PaaSs), or software-as-a-services (SaaSs). The cloud architecture of the computing devices may be embodied according to one of a plurality of different configurations. For instance, if configured according to MICROSOFT AZURE^{™}, roles are provided, which are discrete scalable components built with managed code. Worker roles are for generalized development and may perform background processing for a web role. Web roles provide a web server and listen for and respond to web requests via an HTTP (hypertext transfer protocol) or HTTPS (HTTP secure) endpoint. VM roles are instantiated according to tenant defined configurations (e.g., resources, guest operating system). Operating system and VM updates are managed by the cloud. A web role and a worker role run in a VM role, which is a virtual machine under the control of the tenant. Storage and SQL services are available to be used by the roles.

In some embodiments, the computing devices may be configured into multiple, logically-grouped servers (run on server devices), referred to as a server farm. The computing devices may be geographically dispersed, administered as a single entity, or distributed among a plurality of server farms. The computing devices within each farm may be heterogeneous. One or more of the computing devices may operate according to one type of operating system platform (e.g., WINDOWS NT, manufactured by Microsoft Corp. of Redmond, Wash.), while one or more of the computing devices may operate according to another type of operating system platform (e.g., Unix or Linux). The computing devices may be logically grouped as a farm that may be interconnected using a wide-area network (WAN) connection or medium-area network (MAN) connection. The computing devices may each be referred to as, and operate according to, a file server device, application server device, web server device, proxy server device, or gateway server device.

Note that cooperation between devices (e.g., clinician computing devices) of other networks and the devices of the cloud (and/or cooperation among devices of the cloud) may be facilitated (or enabled) through the use of one or more application programming interfaces (APIs) that may define one or more parameters that are passed between a calling application and other software code such as an operating system, library routine, and/or function that provides a service, that provides data, or that performs an operation or a computation. The API may be implemented as one or more calls in program code that send or receive one or more parameters through a parameter list or other structure based on a call convention defined in an API specification document. A parameter may be a constant, a key, a data structure, an object, an object class, a variable, a data type, a pointer, an array, a list, or another call. API calls and parameters may be implemented in any programming language. The programming language may define the vocabulary and calling convention that a programmer employs to access functions supporting the API. In some implementations, an API call may report to an application the capabilities of a device running the application, including input capability, output capability, processing capability, power capability, and communications capability.

As should be appreciated by one having ordinary skill in the art, one or more computing devices of the cloud platform (or other platform types), as well as of other networks communicating with the cloud platform, may be embodied as an application server, computer, among other computing devices. In that respect, one or more of the computing devices comprises one or more processors, input/output (I/O) interface(s), one or more user interfaces (UI), which may include one or more of a keyboard, mouse, microphone, speaker, tactile device (e.g., comprising a vibratory motor), touch screen displays, etc., and memory, all coupled to one or more data busses.

The memory may include any one or a combination of volatile memory elements (e.g., random-access memory RAM, such as DRAM, and SRAM, etc.) and nonvolatile memory elements (e.g., ROM, Flash, solid state, EPROM, EEPROM, hard drive, tape, CDROM, etc.). The memory may store a native operating system, one or more native applications, emulation systems, or emulated applications for any of a variety of operating systems and/or emulated hardware platforms, emulated operating systems, etc. In some embodiments, a separate storage device may be coupled to the data bus or as a network-connected device. The storage device may be embodied as persistent memory (e.g., optical, magnetic, and/or semiconductor memory and associated drives). The memory comprises an operating system (OS) and application software, including the analytics application described herein.

The computer-readable storage medium may be referred to as a computer-readable non-transitory storage medium. The computer-readable storage medium may also be referred to as a tangible computer readable medium. A computer readable signal medium may include a propagated data signal with computer executable code embodied therein, for example, in baseband or as part of a carrier wave. 'Computer memory' or 'memory' is an example of a computer-readable storage medium. 'Controller' may be any device (e.g., microchip) that is configured to perform the control operations described in the present application.

Execution of the software may be implemented by one or more processors under the management and/or control of the operating system. The processor may be embodied as a custom-made or commercially available processor, a central processing unit (CPU) or an auxiliary processor among several processors, a semiconductor based microprocessor (in the form of a microchip), a macroprocessor, one or more application specific integrated circuits (ASICs), a plurality of suitably configured digital logic gates, and/or other well-known electrical configurations comprising discrete elements both individually and in various combinations to coordinate the overall operation of the computing device.

When certain embodiments of the computing device are implemented at least in part with software (including firmware), it should be noted that the software may be stored on a variety of non-transitory computer-readable (storage) medium for use by, or in connection with, a variety of computer-related systems or methods. In the context of this document, a computer-readable storage medium may comprise an electronic, magnetic, optical, or other physical device or apparatus that may contain or store a computer program (e.g., executable code or instructions) for use by or in connection with a computer-related system or method. The software may be embedded in a variety of computer-readable storage mediums for use by, or in connection with, an instruction execution system, apparatus, or device, such as a computer-based system, processor-containing system, or other system that can fetch the instructions from the instruction execution system, apparatus, or device and execute the instructions.

When certain embodiments of the computing device are implemented at least in part with hardware, such functionality may be implemented with any or a combination of the following technologies, which are all well-known in the art: a discreet logic circuit(s) having logic gates for implementing logic functions upon data signals, an application specific integrated circuit (ASIC) having appropriate combinational logic gates, a programmable gate array(s) (PGA), a field programmable gate array (FPGA), relays, contactors, etc. While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: passive medical sensing device
- 110: housing
- 120: magneto-mechanical resonator
- 121: movable magnet
- 122: restoring unit
- 123: restoring magnet
- 130: rotational axis
- 140: termination magnet
- 150: collector of soft magnetic material
- 200: arrangement
- 210: probe
- 220: emitter
- 230: receiver
- 240: controller

## Claims

1. A passive medical sensing device (100) for at least one of sensing or position tracking, the device (100) comprising:
a housing (110), and
a plurality of magneto-mechanical resonators (120) arranged in the housing (110),
wherein at least one of the plurality of magneto-mechanical resonators (120) is magnetically coupled to at least another of the plurality of magneto-mechanical resonators (120), and
wherein at least one of the magneto-mechanical resonators (120) comprises:
at least one movable magnet (121) mounted to the housing (110) and configured to rotate around a rotational axis (130), and
a restoring unit (122) configured for exerting a restoring force on the at least one movable magnet (121) when the movable magnet (121) is deflected from a rest position of the movable magnet (121) with respect to the rotational axis (130).

2. The device (100) according to claim 1, wherein the restoring unit (122) is a restoring magnet (123).

3. The device (100) according to claim 2, wherein the restoring magnet (123) is fixed to the housing (110) such that no rotational movement of the restoring magnet (123) is allowed.

4. The device (100) according to any of claims 2 or 3, wherein a magnetic moment of each of the restoring magnets (123) of the plurality of magneto-mechanical resonators (120) is arranged in parallel with a magnetic moment of a restoring magnet (123) of a respective neighboring magneto-mechanical resonator (120), and wherein a magnetic moment of each of the movable magnets (121) of the plurality of magneto-mechanical resonators (120) is arranged in parallel with a magnetic moment of a movable magnet (121) of the respective neighboring magneto-mechanical resonator (120) when the movable magnets (121) are in their respective rest position.

5. The device (100) according to any of claims 2 or 3, wherein a magnetic moment of each of the restoring magnets (123) of the plurality of magneto-mechanical resonators (120) is arranged antiparallel to a magnetic moment of a restoring magnet (123) of a respective neighboring magneto-mechanical resonator (120), and wherein a magnetic moment of each of the movable magnets (121) of the plurality of magneto-mechanical resonators (120) is arranged antiparallel to a magnetic moment of a movable magnet (121) of the respective neighboring magneto-mechanical resonator (120) when the movable magnets (121) are in their respective rest position.

6. The device (100) according to any of the preceding claims, wherein the movable magnet (121) is located on the rotational axis (130), and/or wherein the rotational axis (130) is perpendicular to a magnetic dipole moment of the movable magnet (121).

7. The device (100) according to any of the preceding claims, wherein each of the plurality of magneto-mechanical resonators (120), when the movable magnet (121) oscillates around the rest position, is configured to effect a change of the magnetic field at the position of the movable magnet (121) of the at least another of the plurality of magneto-mechanical resonators (120) in a direction perpendicular to both the rotational axis (130) and the magnetic dipole moment of the movable magnet (121) of the at least another of the plurality of magneto-mechanical resonators (120) that is larger than 1 µT, preferably larger than 10 µT, more preferably larger than 100 µT, thus magnetically coupling the magneto-mechanical resonator (120) with the at least another of the plurality of magneto-mechanical resonators (120).

8. The device (100) according to claim 7, wherein there are two frequencies: f of the device (100) and resonance frequency f₀ of the at least one of the plurality of the plurality of the magneto-mechanical resonators (120), and a resonance frequency f of the device (100) differs from the resonance frequency f₀ of the plurality of magneto-mechanical resonators (120).

9. The device (100) according to any of the preceding claims, wherein a length of the movable magnets (121) of the plurality of magneto-mechanical resonators (120) in the direction of the rotational axis (130) is larger than a length of the movable magnets (121) perpendicular to the rotational axis (130), and/or wherein each of the of the plurality of magneto-mechanical resonators (120) comprises a collector of a soft magnetic material (150) arranged adjacent to the movable magnet (121) and configured for focusing a magnetic field applied to or emitted from the movable magnet (121).

10. The device (100) according to any of the preceding claims, wherein the plurality of magneto-mechanical resonators (120) is arranged in a one-dimensional, a two-dimensional or a three-dimensional grid.

11. The device (100) according to any of the preceding claims, wherein the device (100) comprises a termination magnet (140) attached to the housing (110) and configured for correcting edge effects of the plurality of magneto-mechanical resonators (120), and/or wherein at least one of the of the plurality of magneto-mechanical resonators (120) that is arranged at a border area of the of the plurality of magneto-mechanical resonators (120) comprises a movable magnet (121) that is spatially fixed in order to correct for edge effects of the plurality of magneto-mechanical resonators (120).

12. An arrangement (200) comprising
a probe (210), the probe comprising a device (100) according to any of claims 1 to 11,
an emitter (220), configured for emitting a pulse of an electro-magnetic radiation for deflecting the movable magnets (121) of the plurality of magneto-mechanical resonators (120) of the device (100) out of the rest position of the movable magnets (121) and for exciting an oscillating movement of the movable magnets (121) with a resonance frequency f of the device (100),
a receiver (230), configured for receiving an electro-magnetic field emitted by the movable magnets (121) of the magneto-mechanical resonators (120) of the device (100) oscillating with the resonance frequency f, and
a controller (240), configured for determining a position and/or an orientation of the device (100) based on the received electro-magnetic field emitted by the movable magnets (121) of the magneto-mechanical resonators (120) of the device (100) oscillating with the resonance frequency f.

13. Use of an arrangement (200) according to claim 12 for determining a position and/or an orientation of a probe (210) comprising a device (100) according to any of claims 1 to 11 inserted into a subject.

14. Method of sensing or tracking of a position of a probe (210) comprising a passive medical sensing device (100) according to any of claims 1 to 11, the method comprising the steps of:
providing a probe (210) comprising the passive medical sensing device (100) according to any of claims 1 to 11;
inserting the probe (210) into an object;
emitting a pulse of an electro-magnetic radiation for deflecting the movable magnets (121) of the plurality of magneto-mechanical resonators (120) of the device (100) out of the rest position of the movable magnets (121) and for exciting an oscillating movement of the movable magnets (121) with a resonance frequency f of the device (100);
receiving an electro-magnetic field emitted by the movable magnets (121) of the magneto-mechanical resonators (120) of the device (100) oscillating with the resonance frequency f; and
determining a position and/or an orientation of the device (100) based on the received electro-magnetic field emitted by the movable magnets (121) of the magneto-mechanical resonators (120) of the device (100) oscillating with the resonance frequency f.

15. A computer program comprising instructions which, when the program is executed by a computing device, cause the computing device to carry out the steps of the method according to claim 14.
